# DEMANDE DE BREVET EUROPEEN

(11) **EP 0 676 401 A1**
(43) Date de publication de la demande: **11.10.1995**
(21) Numéro de dépôt: 95400754.8
(22) Date de dépôt: 05.04.1995
(51) Int. Cl.: C07D 493/10, C07D 491/107, C07D 311/96, G03C 1/685

(54) **Spiro fluorène- 2H -benzopyranes annélés et leur utilisation dans le domaine de l'optique ophtalmique**

(30) Priorité: 06.04.1994 FR 9404028
(71) Demandeur: ESSILOR INTERNATIONAL (COMPAGNIE GENERALE D'OPTIQUE), F-94227 Charenton cédex (FR)
(72) Inventeur: Pozzo, Jean Luc, F-13008 Marseille (FR); Guglielmetti, Robert, Boulevard des Alisiers,F-13009 Marseille (FR); Samat, André, F-13013 Marseille (FR); Lokshin, Vladimir, F-13009 Marseille (FR); Harie, Guenaelle, F-13008 Marseille (FR)
(74) Mandataire: Casalonga, Axel

(57) **Abrégé**

L'invention est relative à des composés photochromiques de formule générale : 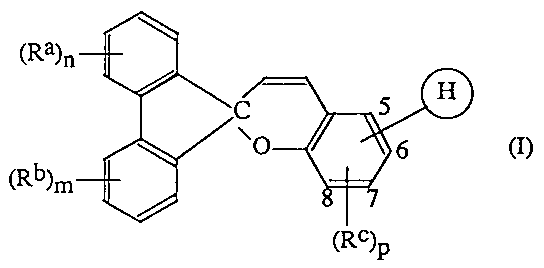
dans laquelle R^{a}, R^{b} et R^{c} désignent hydrogène; alkyle; aryle; OR, SR, COR ou COOR, dans lesquels R désigne hydrogène, alkyle ou aryle, amino de formule NR₁R₂, dans lequel R¹ et R₂ désignent hydrogène, alkyle, cycloalkyle, aryle, R₁ et R₂ pouvant former avec l'atome d'azote, un hétérocycle comportant 4 à 7 chaînons et pouvant contenir en plus un ou plusieurs hétéroatomes choisis parmi l'azote, l'oxygène, le soufre, un atome d'halogène; un groupement mono ou polyhaloalkyle; un groupement NO₂, CN ou SCN; n et m désignent des nombres entiers de 1 à 4 suivant le nombre de substitutions sur le noyau et p peut être égal à 1 ou 2 suivant le nombre de substitutions sur le noyau; H est un hétérocycle aromatique condensé en 5,6 ou 6,7, ayant 5 ou 6 chaînons comportant un ou plusieurs hétéroatomes choisis parmi l'azote, le selenium, l'oxygène, le soufre ou un carbocycle aromatique à 5-6 chaînons condensé en 7,8; ces cycles pouvant être substitués par un ou plusieurs groupements alkyle, alcoxy, amino, aryle, aralkyle ou condensés avec un noyau aromatique, et à leur utilisation dans l'optique ophtalmique.

## Description

L'invention a pour objet de nouveaux composés photochromiques, plus particulièrement des composés photochromiques comportant dans leur formule chimique un noyau de la famille des chromènes avec un carbone sp³ spiranique comportant un noyau fluorène, ainsi que leur utilisation dans le domaine ophtalmique, en particulier dans et/ou sur des lentilles ophtalmiques.

Le photochromisme est un phénomène connu depuis de nombreuses années. On dit qu'un composé est photochromique lorsque ce composé, irradié par un faisceau lumineux, dont certaines longueurs d'onde se situent dans le domaine de l'ultraviolet, change de couleur et revient à sa couleur originelle dès que les radiations cessent.

Les applications de ce phénomène sont multiples, mais une des applications connues plus particulièrement intéressante concerne le domaine de l'optique ophtalmique.

De tels composés sont utilisables dans la fabrication de lentilles ou verres pour lunettes, en vue de filtrer les radiations lumineuses en fonction de leur intensité.

L'incorporation des composés photochromiques dans un matériau organique constituant une lentille ophtalmique, permet d'obtenir un verre dont le poids est considérablement réduit par rapport aux lentilles classiques en verre minéral qui comportent des halogénures d'argent à titre d'agent photochrome. Leur incorporation dans des matériaux organiques a toujours posé des difficultés techniques.

Toutefois, tout composé à propriété photochromique n'est pas forcément utilisable dans le domaine de l'optique ophtalmique. En effet, le composé photochromique doit répondre à un certain nombre de critères, dont entre autres :
- une forte colorabilité qui est la mesure de la capacité pour un composé photochromique de présenter une couleur intense après isomérisation;
- une coloration après absorption de la lumière rendant apte le composé photochromique, seul ou en combinaison avec d'autres composés photochromiques à être utilisé dans des verres ou lentilles ophtalmiques;
- une absence de coloration ou très faible coloration sous la forme initiale;
- une cinétique rapide de coloration ou de décoloration;
- un photochromisme se manifestant dans une plage de température la plus large possible, et en particulier de préférence entre 0 et 40°C.

Les composés photochromiques organiques connus et utilisés actuellement, présentent généralement un photochromisme décroissant lorsque la température augmente, de sorte que le photochromisme est particulièrement marqué à des températures proches de 0°C, alors qu'il est beaucoup plus faible, voire inexistant, à des températures de l'ordre de 40°C qui sont des températures que peuvent atteindre les verres lors notamment de l'exposition au soleil.

Un autre problème rencontré pour les composés photochromiques de l'état de la technique est leur durée de vie. On constate, en effet, pour certains produits de l'état de la technique, une durée de vie relativement réduite. En effet, après un certain nombre de cycles de coloration et de décoloration, le composé photochromique se transforme généralement en produits d'oxydation et ne présente plus les propriétés photochromes réversibles.

De nombreux composés photochromiques de type chroménique ont déjà été synthétisés. Par exemple, la demande de brevet EP 246 114 décrit une série de composés photochromiques dans lesquels un groupe adamantane est introduit en position 2 du noyau benzopyrane ou naphtopyrane, ou encore la demande de brevet WO 90/07507 où deux groupes cyclopropyle sont attachés à la position 2 du composé cyclique de type benzopyrane ou naphtopyrane. On peut encore citer, du même inventeur, la demande de brevet WO 91/00861 où un groupe norcamphore ou un groupe tricyclodécane est introduit en position 2 de composés photochromiques du même type.

On a déjà décrit dans le brevet US-3.567.605 des dérivés photochromiques de type benzopyrane et naphtopyrane substitués en position 2 du cycle pyrannique. Ces composés présentent cependant des constantes cinétiques de décoloration relativement faibles.

On connaît, par ailleurs, dans la demande EP-A-0401958, des dérivés photochromes présentant également des constantes de cinétique de décoloration faibles et moins bien adaptées à l'application envisagée.

La société demanderesse a proposé dans sa demande de brevet français n° 2.688.782, des composés photochromiques hétérocycliques benzopyranes comprenant deux groupes phényle, en position 2 du cyle benzopyrane.

Ces composés présentent une bonne colorabilité dans le domaine rouge qui leur permet d'être utilisés dans l'optique ophtalmique avec des composés photochromiques donnant une couleur bleue, en vue d'obtenir une coloration finale naturelle lors de l'exposition à la lumière. Ils présentent, par ailleurs, une absence de coloration ou une très faible coloration à l'état initial et une cinétique rapide de coloration et de décoloration à des températures de l'ordre de 0 à 40°C.

La demanderesse a découvert, de manière surprenante, une nouvelle famille de chromènes hétérocycliques comportant un carbone spiranique relié à un noyau fluorène, présentant des propriétés photochromiques particulièrement intéressantes. Les composés du type spiro-fluorène, conformes à l'invention, présentent une colorabilité dans le domaine du rouge plus élevée que celle des photochromiques du type 2,2-diphényl-[2H]chromènes cités ci-dessus.

La demanderesse a en effet découvert que le groupement fluorène conduisait à un déplacement dit bathochromique (vers les longueurs d'onde du rouge) du spectre d'absorption de la forme colorée par rapport à ceux correspondant aux composés 2,2-diphényl-[2H]chromènes décrits précédemment.

Les composés conformes à l'invention peuvent, par ces propriétés, être utilisés dans l'optique ophtalmique avec des composés photochromiques donnant une couleur bleue, dans des quantités sensiblement plus faibles, en vue d'obtenir une coloration finale naturelle lors de l'exposition à la lumière.

Les composés conformes à l'invention présentent par ailleurs une absence de coloration ou une très faible coloration à l'état initial et une cinétique rapide de coloration ou de décoloration dans une plage à température très large, comprise en particulier entre 0 et 40°C.

La demanderesse a également constaté que ces composés avaient une durée de vie particulièrement longue.

Toutes ces propriétés font que ces nouveaux composés photochromiques sont particulièrement intéressants dans leur utilisation dans l'optique ophtalmique, et en particulier pour leur utilisation dans et/ou sur des lentilles ophtalmiques.

On appelle lentilles ophtalmiques, au sens de l'invention, des verres de lunettes, en particulier des verres solaires, des lentilles de contact.

Un objet de l'invention est donc constitué par les nouveaux composés photochromiques.

Un autre objet de l'invention est constitué par leur utilisation dans l'optique ophtalmique.

L'invention a également pour objet des compositions destinées à être utilisées pour le revêtement des lentilles ophtalmiques ou leur incorporation dans ces lentilles.

D'autres objets de l'invention apparaîtront à la lecture de la description et des exemples qui suivent.

Les composés photochromiques de l'invention sont essentiellement caractérisés par le fait qu'ils répondent à la formule générale (I) : 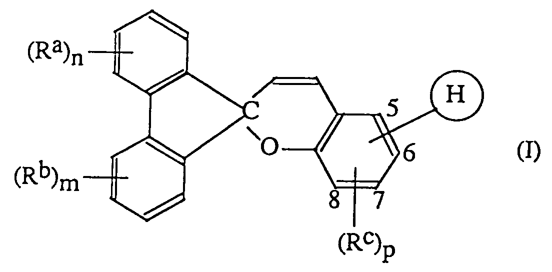
dans laquelle R^{a}, R^{b} et R^{c} désignent, indépendamment l'un de l'autre, un atome d'hydrogène, un groupement alkyle, un groupement aryle, un groupement OR, SR, COR ou COOR, dans lesquels R désigne un atome d'hydrogène, un groupement alkyle ou un groupement aryle, un groupement amino de formule NR₁R₂, dans lequel R₁ et R₂ désignent, indépendamment l'un de l'autre, un atome d'hydrogène, un groupement alkyle, un groupement cycloalkyle, un groupement aryle, R₁ et R₂ pouvant former avec l'atome d'azote, un hétérocycle comportant 4 à 7 chaînons et pouvant contenir en plus un ou plusieurs hétéroatomes choisis parmi l'azote, l'oxygène, le soufre, un atome d'halogène, un groupement mono ou polyhaloalkyle; un groupement NO₂, CN ou SCN; n et m désignent des nombres entiers de 1 à 4 suivant le nombre de substitutions sur le noyau et p peut être égal à 1 ou 2 suivant le nombre de substitutions sur le noyau; les groupements R^{a}, R^{b}, R^{c} peuvent avoir des significations différentes lorsque m, n et p sont supérieurs à 1 et suivant la position sur les noyaux; H est un hétérocycle aromatique à S ou 6 chaînons, condensé en 5,6 ou 6,7, comportant un ou plusieurs hétéroatomes choisis parmi l'oxygène, le selenium, le soufre ou l'azote ou un carbocycle aromatique à 5 ou 6 chaînons, condensé en 7,8; ledit carbocycle ou hétérocycle H pouvant être substitué par un ou plusieurs groupements alkyle, alcoxy, amino, aryle, aralkyle ou condensé avec un autre cycle de 5 à 10 chaînons.

Dans la formule (I) précitée, un groupement alkyle désigne de préférence un groupement ayant 1 à 6 atomes de carbone; un groupement cycloalkyle désigne de préférence un groupement ayant de 3 à 7 atomes de carbone; le groupe aryle désigne de préférence phényle; halogène désigne de préférence chlore, brome, fluor; le groupement polyhaloalkyle désigne de préférence le groupe CF₃.

Les noyaux hétérocycliques H condensés en position 5,6 ou 6,7 sur le noyau benzopyrane, sont représentés plus particulièrement par l'une des formules (II) ou (III) : 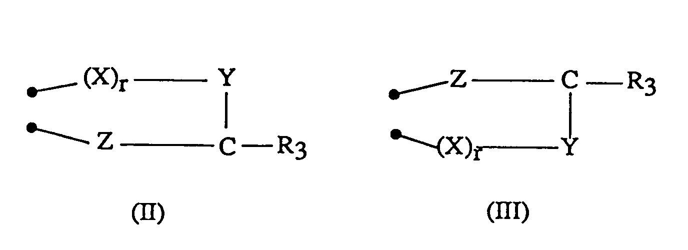
dans lesquelles :
X désigne N ou le groupe CR₄, l'atome d'azote ou de carbone étant relié à l'atome adjacent par une double liaison pour assurer l'aromaticité de l'hétérocycle condensé sur le noyau benzopyrane de la formule (I); r vaut 0 ou 1;
Y et Z, indépendamment l'un de l'autre, désignent NR₅, S, O, Se; ou bien N ou CR₆, chacun lié à l'atome adjacent par une double liaison pour assurer l'aromaticité de l'hétérocycle condensé sur le noyau benzopyrane.
R₅ et R₆, désignent indépendamment l'un de l'autre, un atome d'hydrogène, un alkyle en C₁-C₆ ou un phényle;
CR₃ est lié à un atome adjacent par une double liaison de manière à assurer l'aromaticité de l'hétérocycle condensé sur le noyau benzopyrane;
R₃ désigne un atome d'hydrogène, un alkyle en C₁-C₆, un phényle ou forme avec l'un des groupes R₅ ou R₆ un cycle C, aromatique ou non, ayant de 5 à 10 chaînons, de préférence de 6 à 7 chaînons, éventuellement substitué par un ou plusieurs radicaux R^{a} tels que définis ci-dessus.
l'un des groupements X, Y et Z désigne N, O, S, Se ou NR₅.

Dans les formules précitées (II) ou (III), les cycles C préférentiels sont choisis parmi benzène, cyclohexane ou cycloheptane.

Parmi les hétérocycles H préférentiels de formule (II) ou (III), on peut citer les noyaux pyrimidiniques, pyraziniques, pyridimiques, oxazoliques, pyrroliques, thiazoliques éventuellement substitués, ou bien les furanes condensés par un cycle tels que le benzofurane, le cycloheptanofurane ou le cyclohexanofurane.

Les composés spiro[fluorène-chromènes] de la présente invention présentent une plus forte colorabilité dans le domaine du rouge que leurs homologues 2,2-diphényl-[2H]chromènes. Le noyau fluorène induit, par rapport au groupement diphényle, un déplacement bathochromique de la bande correspondant au maximum d'absorption (λ₁) de l'ordre de +10 à +30 nm.

Une famille de composés spiro[fluorène-chromènes] de l'invention particulièrement intéressante, est constituée par les composés de formule (I), dans laquelle H est un hétérocycle comportant 5 chaînons et est condensé en position 5,6 ou 6,7 sur le cycle benzopyrane. Ces composés particuliers présentent un spectre d'absorption de la forme colorée faisant apparaître une nouvelle bande correspondant à un pic d'absorption (λ₃) de l'ordre de +500 nm.

Parmi ces composés de cette famille, ceux de formule (I) pour lesquels l'hétérocycle est condensé en position 6,7, sont plus particulièrement préférés, du fait que l'annélation de l'hétérocycle H en position 6,7 induit un élargissement de la bande principale correspondant au (λ₁) avec l'apparition d'un second maximum d'absorption (λ₂). De plus, l'annélation en position 6,7 de l'hétérocycle H, produit un déplacement de la deuxième bande d'absorption (λ₃) de +15 à 30 nm. On obtient ainsi des composés chromènes dont la coloration, à l'état irradié, est déplacée davantage vers le rouge et dont l'intensité est sensiblement plus importante.

A titre d'exemples de composés de formule (I) utilisés selon la présente invention, on peut citer ceux répondant aux formules chimiques suivantes :

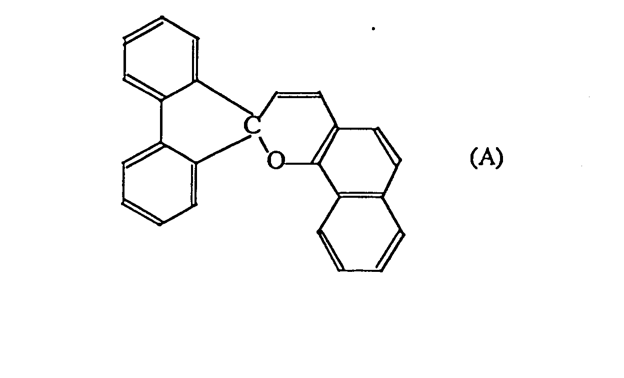

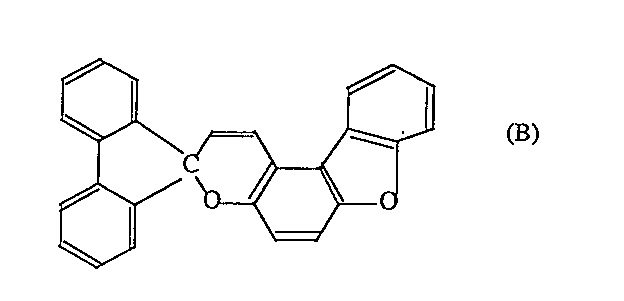

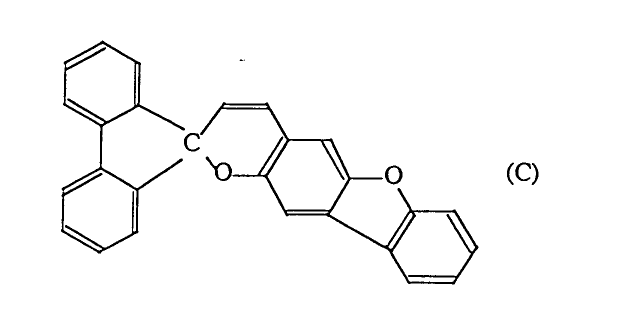

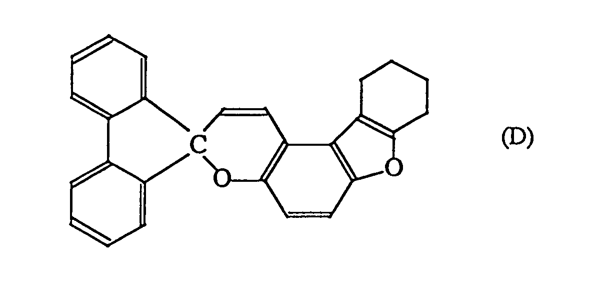

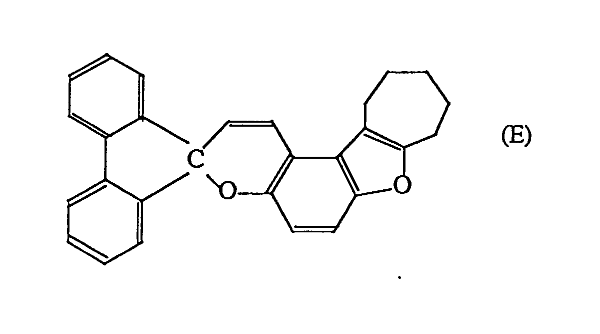

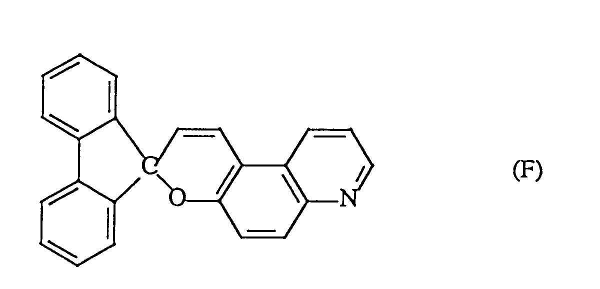

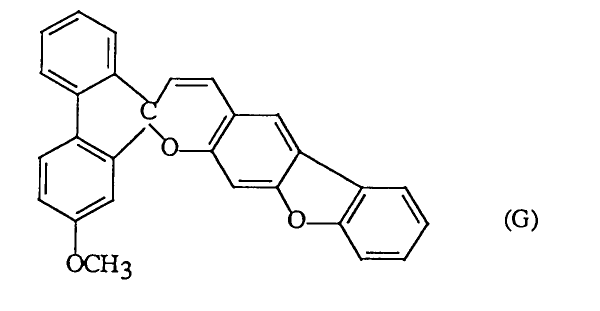

Les composés conformes à l'invention peuvent être préparés selon le schéma réactionnel suivant :

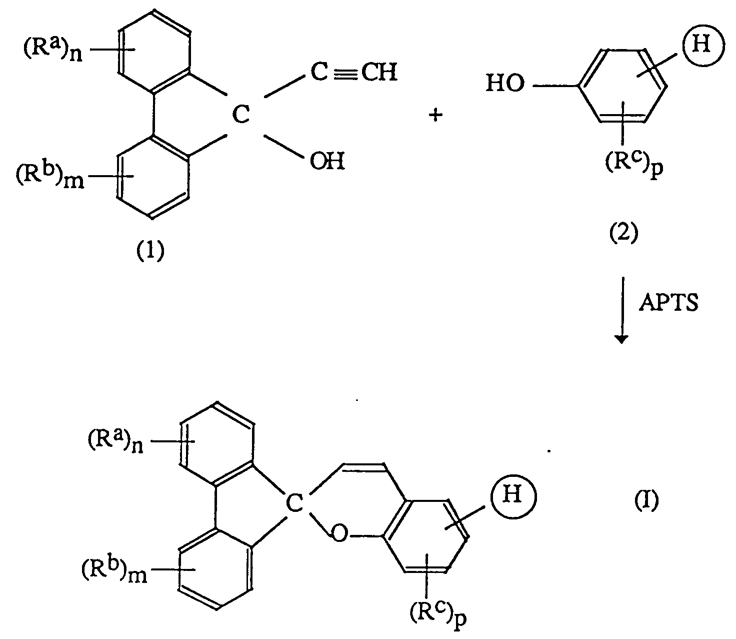

Selon ce schéma réactionnel, on fait réagir un dérivé du 9-éthynyl 9-fluorénol de formule (1), dans laquelle R^{a}, R^{b}, m et n ont les mêmes significations indiquées ci-dessus sur un phénol de formule (2) dans laquelle R^{c}, p et H ont les mêmes significations indiquées ci-dessus, en présence de l'acide p-toluènesulfonique (APTS).

Les composés de l'invention peuvent être également préparés selon le schéma réactionnel suivant :

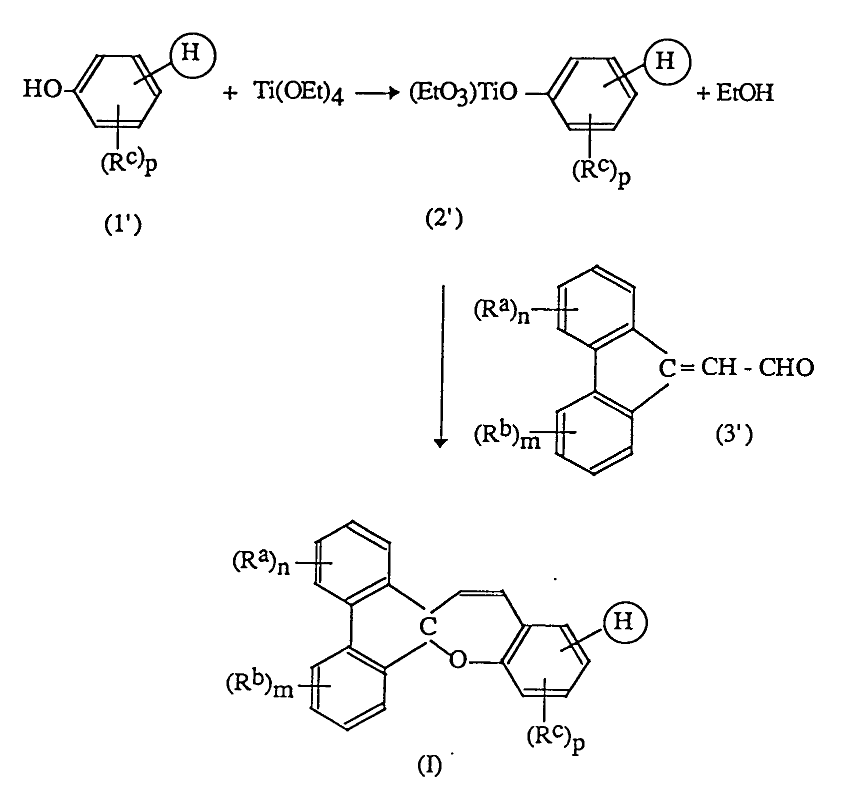

Selon ce schéma réactionnel, dans les formules (1'), (2') et (3') ci-dessus, les radicaux R^{a}, R^{b}, R^{c} et m, n et p ont les mêmes significations indiquées précédemment.

Les composés photochromiques conformes à l'invention peuvent être utilisés pour réaliser des lentilles ophtalmiques photochromiques.

Les composés conformes à l'invention peuvent être introduits dans une composition destinée à être appliquée sur ou être introduite dans un matériau polymère organique transparent pour obtenir un article transparent photochromique. Ils peuvent également être introduits dans des compositions solides telles que films plastiques, plaques et lentilles pour réaliser des matériaux utilisables notamment comme lentilles ophtalmiques, lunettes de soleil, viseurs, optiques de caméra et filtres.

Les compositions liquides qui constituent un objet de l'invention sont essentiellement caractérisées par le fait qu'elles contiennent sous forme dissoute ou dispersée les composés conformes à l'invention dans un milieu à base de solvants appropriés pour être appliqués ou introduits dans un matériau polymère transparent.

Des solvants plus particulièrement utilisables sont des solvants organiques choisis parmi le benzène, le toluène, le chloroforme, l'acétate d'éthyle, la méthyléthylcétone, l'acétone, l'alcool éthylique, l'alcool méthylique, l'acétonitrile, le tétrahydrofurane, le dioxane, l'éther méthylique d'éthylèneglycol, le diméthylformamide, le diméthylsulfoxyde, le méthylcellosolve, la morpholine, et l'éthylèneglycol.

Lorsque les composés conformes à l'invention sont dispersés, le milieu peut également contenir de l'eau.

Selon une autre forme de réalisation, les composés conformes à l'invention peuvent être introduits et de préférence dissous dans des solutions incolores ou transparentes préparées à partir de polymères, de copolymères ou de mélanges de polymères transparents dans un solvant organique approprié.

Les exemples de telles solutions sont entre autres des solutions de nitrocellulose dans l'acétonitrile, de polyvinylacétate dans l'acétone, de chlorure de polyvinyle dans la méthyléthylcétone, de polyméthylméthacrylate dans l'acétone, d'acétate de cellulose dans le diméthylformamide, de polyvinylpyrrolidone dans de l'acétonitrile, de polystyrène dans le benzène, d'éthylcellulose dans du chlorure de méthylène.

Ces compositions peuvent être appliquées sur des supports transparents tels qu'en térèphtalate de polyéthylèneglycol, de papier borylé, de triacétate de cellulose et séchées pour obtenir un matériau photochromique qui peut se colorer en présence d'une radiation ultraviolette, et qui retourne à l'état non coloré et transparent en l'absence de la source de radiation.

Les composés photochromiques de la présente invention ou les compositions les contenant définies ci-dessus peuvent être appliqués ou incorporés dans un matériau organique polymérisé transparent solide approprié pour des éléments ophtalmiques tels que des lentilles ophtalmiques ou des matériaux utiles pour être utilisés dans des lunettes de soleil, des viseurs, des optiques de caméras et des filtres.

A titre de matériaux solides transparents qui peuvent être utilisés pour réaliser des lentilles ophtalmiques conformes à l'invention, on peut citer les polymères de polyol(allylcarbonate), des polyacrylates, des poly(alkylacrylate) tels que des polyméthylméthacrylates, l'acétate de cellulose, le triacétate de cellulose, le propionate acétate de cellulose, le butyrate acétate de cellulose, le poly(vinylacétate), le poly(vinylalcool), les polyuréthanes, les polycarbonates, les polyéthylènetérèphtalates, les polystyrènes, les (polystyrène-méthylméthacrylate), les copolymères de styrène et d'acrylonitrile, les polyvinylbutyrates.

Les copolymères transparents ou des mélanges de polymères transparents sont également appropriés pour réaliser de tels matériaux.

On peut citer à ce sujet les matériaux préparés à partir de polycarbonates tels que le poly(4,4'-dioxydiphénol-2,2 propane), le polyméthylméthacrylate, les polyol(allylcarbonate) tels qu'en particulier le diéthylèneglycol bis(allylcarbonate) et ses copolymères tels que par exemple avec l'acétate de vinyle. On peut citer en particulier les copolymères de diéthylèneglycol bis(allylcarbonate) et d'acétate de vinyle (80-90/10-20) et encore le copolymère de diéthylèneglycol bis(allylcarbonate) avec l'acétate de vinyle, l'acétate de cellulose et le propionate de cellulose, le butyrate de cellulose (80-85/15-20).

Les polyols(allylcarbonate) sont préparés en utilisant des allylcarbonates de polyols liquides aliphatiques ou aromatiques, linéaires ou ramifiés tels que les glycols aliphatiques de bis-allylcarbonate ou les alkylène bis(allylcarbonate). Parmi les polyols (allylcarbonate) qui peuvent être utilisés pour préparer les matériaux transparents solides utilisables conformément à l'invention, on peut citer l'éthylèneglycol bis(allylcarbonate), le diéthylèneglycol bis(2-méthallylcarbonate), le diéthylèneglycol bis(allylcarbonate), l'éthylèneglycol bis(2-chloroallylcarbonate), le triéthylèneglycol bis(allylcarbonate), le 1,3-propanediol bis(allylcarbonate), le propylèneglycol bis(2-éthylallylcarbonate), le 1,3-butanediol bis(allylcarbonate), le 1,4-butanediol bis(2-bromoallylcarbonate), le dipropylèneglycol bis(allylcarbonate), le triméthylèneglycol bis(2-éthylallylcarbonate), le pentaméthylène glycol bis(allylcarbonate), l'isopropylène bisphénol bis (allyl carbonate). Le produit le plus important est constitué par le diéthylène glycol bis(allylcarbonate) encore connu sous la dénomination CR39.

La quantité de composés photochromiques à utiliser conformément à l'invention, soit dans la composition, soit au moment de son introduction dans le support solide, n'est pas critique et dépend généralement de l'intensité de la couleur que la composition peut conférer au matériau après exposition aux radiations. D'une manière générale, plus on ajoute de composés photochromiques, plus la coloration sous irradiation sera importante.

Conformément à l'invention, on utilise une quantité suffisante pour conférer au matériau traité la propriété de changer de couleur au moment de l'exposition à la radiation. Cette quantité de composés photochromiques est généralement comprise entre 0,01 et 20% en poids et de préférence entre 0,05 et 10% en poids par rapport au poids total du matériau optique ou de la composition.

Les composés photochromiques conformes à l'invention peuvent également être introduits dans un support temporaire (tel qu'un vernis formant un revêtement sur un substrat) de transfert et être transférés ensuite thermiquement dans le substrat comme décrit en particulier dans le brevet US-4.286.957 ou US-4.880.667.

Ces composés peuvent être utilisés avec d'autres composés photochromiques, tels que des composés photochromiques donnant lieu à des colorations différentes telles que bleu, verte, connus dans l'état de la technique. C'est ainsi qu'on peut utiliser des spiro(indoline-oxazines) bien connus dans l'état de la technique.

Une fois appliqués sur des matériaux ophtalmiques ou introduits dans de tels matériaux, on constate après exposition aux irradiations UV, l'apparition d'une coloration et le retour à la couleur ou à la transparence originelle lorsqu'on interrompt l'exposition aux radiations UV.

Les composés conformes à l'invention présentent l'intérêt de permettre ce changement de coloration un grand nombre de fois et ceci à des températures très variables comprises entre 0 et 40°C.

Les exemples suivants sont destinés à illustrer l'invention sans pour autant présenter un caractère limitatif.

### EXEMPLES

### EXEMPLE 1

### Synthèse du spiro[fluorène-9,2'-[2H]naphto[1,2-b]-pyrane]

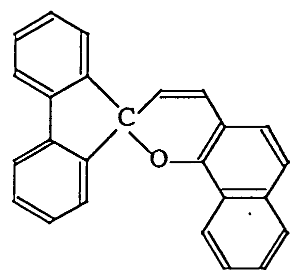

3,09 g de 9-éthynyl-9-fluorénol (15 mmoles) et 2,49 g de 1-naphtol (17,25 mmoles) sont dissous à reflux dans 30 ml de toluène anhydre, sous atmosphère inerte. On additionne ensuite 0,03 g d'APTS. Le mélange réactionnel est laissé à reflux, sous atmosphère inerte, pendant 2 h 30 minutes. Revenu à température ambiante, le mélange est versé sur une solution aqueuse de soude à 10%. Après décantation, la phase aqueuse est extraite en continu au dichlorométhane. Les phases organiques sont réunies puis séchées sur sulfate de magnésium. Le solvant est chassé sous pression réduite. Le composé est précipité par l'hexane puis recristallisé dans le toluène.

| | |
|---|---|
| Rendement | 22% |
| Masse moléculaire | 333,41 g |
| Point de fusion | 137°C |

### EXEMPLE 2

### Synthèse du spiro[3H-benzofurano[3,2-g]chromène-3,9'-fluorène]

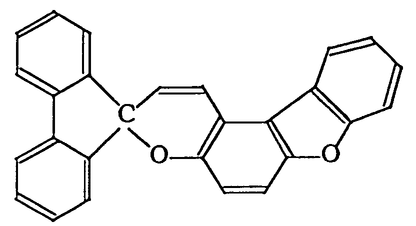

1,03 g de 9-éthynyl-9-fluorénol (5 mmoles) et 1,06 g de 2-hydroxy dibenzofurane (5,75 mmoles) sont dissous à reflux dans 15 ml de toluène anhydre, sous atmosphère inerte. On additionne ensuite 0,01 g d'APTS. Le mélange réactionnel est laissé à reflux, sous atmosphère inerte, pendant 2 heures. Revenu à température ambiante, le mélange est versé sur une solution aqueuse de soude à 10%. Après décantation, la phase aqueuse est extraite en continu au dichlorométhane. Les phases organiques sont réunies puis séchées sur sulfate de magnésium. Le solvant est alors chassé sous pression réduite. Le composé est purifié par chromatographie flash, avec comme mélange éluant 99% pentane-1% éther éthylique. Le solide obtenu après avoir chassé le solvant sous pression réduite, est alors lavé à l'hexane.

| | |
|---|---|
| Rendement | 41% |
| Masse molaire | 372,43 g |
| Point de fusion | 151°C |

### EXEMPLE 3

### Synthèse du spiro[7H-benzofurano[3,2-g]chromène-7,9'-fluorène]

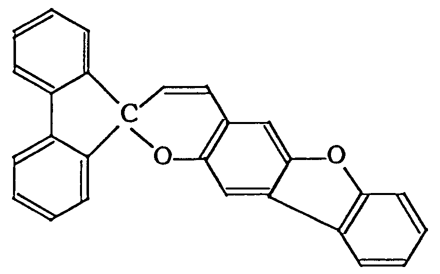

1,03 g de 9-éthynyl-9-fluorénol (5 mmoles) et 1,06 g de 2-hydroxy dibenzofurane (5,75 mmoles) sont dissous à reflux dans 15 ml de toluène anhydre, sous atmosphère inerte. On additionne ensuite 0,01 g d'APTS. Le mélange réactionnel est laissé à reflux, sous atmosphère inerte, pendant 2 heures. Revenu à température ambiante, le mélange est versé sur une solution aqueuse de soude à 10%. Après décantation, la phase aqueuse est extraite en continu au dichlorométhane. Les phases organiques sont réunies puis séchées sur sulfate de magnésium. Le solvant est alors chassé sous pression réduite. Le composé est purifié par chromatographie flash, avec comme mélange éluant 98% pentane-2% éther éthylique, puis recristallisé plusieurs fois dans l'heptane.

| | |
|---|---|
| Rendement | 21% |
| Masse molaire | 372,43 g |
| Point de fusion | 195°C |

### EXEMPLE 4

### Synthèse du 8',9'-tetraméthylène-spiro[fluorène-9,3'-[3H]-furano [3,2-f]chromène]

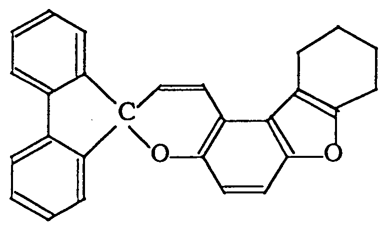

1,44 g de 9-éthynyl-9-fluorénol (7 mmoles) et 1,52 g de 2,3-tétraméthylène-5-hydroxybenzofurane (8,05 mmoles) sont dissous à reflux dans 20 ml de toluène anhydre, sous atmosphère inerte. On additionne ensuite 0,015 g d'APTS. Le mélange réactionnel est laissé à reflux, sous atmosphère inerte, pendant 3 heures. Revenu à température ambiante, le mélange est versé sur une solution aqueuse de soude à 10%. Après décantation, la phase aqueuse est extraite en continu au dichlorométhane. Les phases organiques sont réunies puis séchées sur sulfate de magnésium. Le solvant est alors chassé sous pression réduite. Le composé est purifié par chromatographie flash (100% pentane). Le composé est alors recristallisé dans un mélange hexane-benzène.

| | |
|---|---|
| Rendement | 37% |
| Masse molaire | 376,46 g |
| Point de fusion | 201°C |

### EXEMPLE 5

### Synthèse du 8',9'-pentaméthylène-spiro[fluorène-9,3'-[3H]-furano [3,2-f]chromène]

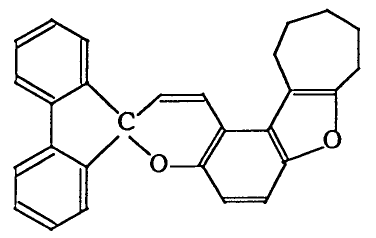

1,44 g de 9-éthynyl-9-fluorénol (7 mmoles) et 1,62 g de 2,3-pentaméthylène-5-hydroxybenzofurane (8. mmoles) sont dissous à reflux dans 20 ml de toluène anhydre, sous atmosphère inerte. On additionne ensuite 0,015 g d'APTS. Le mélange réactionnel est laissé à reflux, sous atmosphère inerte, pendant 3 heures. Revenu à température ambiante, le mélange est versé sur une solution aqueuse de soude à 10%. Après décantation, la phase aqueuse est extraite en continu au dichlorométhane. Les phases organiques sont réunies puis séchées sur sulfate de magnésium. Le solvant est alors chassé sous pression réduite. Le composé est purifié par chromatographie flash (100% pentane). Le composé est alors recristallisé dans un mélange hexane-benzène.

| | |
|---|---|
| Rendement | 41% |
| Masse molaire | 390,49 g |
| Point de fusion | 189°C |

### EXEMPLE 6

### Synthèse du spiro[fluorène-9,8'-[8H]-pyrano [3,2-f]quinoléine]

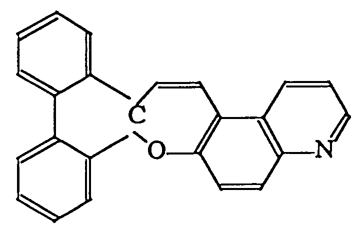

0,5 g de 6-hydroxyquinoléine (3,4 mmoles) sont dissous à température ambiante dans 17 ml de toluène anhydre, sous atmosphère inerte. On additionne au goutte à goutte une solution de 0,79 g de tétraéthyl-orthotitanate (3,4mmoles) dissous dans 4 ml de toluène anhydre. On porte le mélange réactionnel à reflux pendant 30 minutes. Une distillation est effectuée afin d'enlever du milieu l'éthanol formé, le volume recueilli est de 7 ml. On laisse revenir à température ambiante et une solution de 0,8 g de 9-fluorénylidèneacétaldéhyde (3,9 mmoles) dissous dans 14 ml de toluène anhydre est additionnée lentement, sous atmosphère inerte. Le mélange réactionnel est alors porté à reflux pendant 2 heures. Revenu à température ambiante le mélange est versé sur une solution de soude 2M. Après décantation, la phase aqueuse est extraite en continu au dichlorométhane. Les phases organiques sont réunies puis séchées sur sulfate de magnésium. Le solvant est alors chassé sous pression réduite. Le composé est purifié par chromatographie sur colonne (95% dichlorométhane, 5% acétate d'éthyle). Le composé est recristallisé dans le dichlorométhane.

| | |
|---|---|
| Rendement | 62% |
| Masse molaire | 333,39 g |
| Point de fusion | 185°C |

### EXEMPLE 7

### Synthèse du 2'-méthoxy spiro[7H-benzofurano [3,2-g]chromène]-7,9'-fluorène

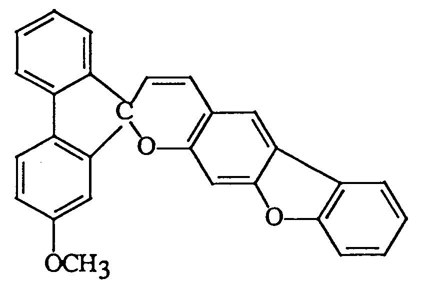

1,77 g de 9-éthynyl 2-méthoxy 9-fluorénol (8 mmoles) et 1,52 g de 2-hydroxy dibenzofurane (8,1 mmoles) sont dissous à 60°C dans 20 ml de toluène anhydre, sous atmosphère inerte. On additionne ensuite 0,019 g d'APTS. Le mélange réactionnel est laissé à 60°C pendant 2 heures, sous atmosphère inerte. Revenu à température ambiante, le mélange est versé sur une solution aqueuse de soude à 10%. Après décantation, la phase aqueuse est extraite en continu au dichlorométhane. Les phases organiques sont réunies puis séchées sur sulfate de magnésium. Le solvant est alors chassé sous pression réduite. Le composé est purifié par chromatographie sur colonne (95% pentane, 5% éther diéthylique). Le composé est recristallisé dans l'heptane.

| | |
|---|---|
| Rendement | 30% |
| Masse molaire | 402,45 g |
| Point de fusion | 176°C |

### ETUDE DES VALEURS SPECTROCINETIQUES DES COMPOSES DES EXEMPLES 1 à 7

On irradie par le rayonnement UV des solutions toluéniques contenant chacune l'un des composés des exemples 1 à 4, à une concentration de 2,5.10⁻⁵ M.

On mesure, d'une part, les colorabilités Aᵢ de chaque solution, après irradiation, correspondant aux maxima d'absorption λᵢ dans le spectre d'absorption de chaque composé.

On mesure également les différents maxima d'absorption (λ₁, λ₂, λ₃) apparaissant dans chaque spectre d'absorption (exprimés en nm).

Les mesures sont effectuées à 25°C (±0,2°C contrôlée par un thermostat extérieur du type Hubert-ministat) dans une cuve en quartz cylin drique de section 10 mm et de longueur optique 10 cm.

On effectue une photolyse des échantillons par des tubes à décharge alimentés par une batterie de capacités
- énergie des éclairs : de l'ordre de 60 J
- durée des éclairs : 50 µs.

A représente la densité optique maximum au temps t, de la solution après éclair, ayant une concentration de composé photochromique dans le toluène de 2,5.10⁻5M.

On mesure enfin les constantes cinétiques k_{Δ} de décoloration thermique (exprimées en s-1) et l'amplitude de décoloration thermique, correspondante (exprimée en pourcentage). On part de la coloration maximale obtenue après irradiation (Aᵢ).

**TABLEAU**

| Exemples de composé | λ1 (nm) | A1 Colorabilité | λ2 (nm) | A2 Colorabilité | λ3 (nm) | A3 Colorabilité | Constantes cinétiques de décoloration (amplitude de décoloration) | |
|---|---|---|---|---|---|---|---|---|
| | | | | | | | k_{Δ1} | k_{Δ2} |
| 1 | 409 | 0,70 | 436 | 0,94 | 495 | 1,44 | <0,001 | |
| 2 | 457 | 0,95 | | | 551 | 0,46 | 0,29 (35%) | 0,06 (65%) |
| 3 | 460 | 1,75 | 491 | 1,82 | 569 | 0,75 | 0,52 (65%) | 0,01 (35%) |
| 4 | 447 | 1,26 | | | 534 | 0,39 | 0,34 (40%) | 0,017 (60%) |
| 5 | 449 | 1,33 | | | 535 | 0,41 | 0,30 (45%) | 0,02 (55%) |
| 6 | 443 | 0,75 | 475 | 0,9 | | | 0,19 (55%) | 0,05 (45%) |
| 7 | 458 | 1,55 | 492 | 1,76 | 566 | 0,70 | 0,66 (45%) | 0,014 (55%) |

### TESTS COMPARATIFS VISUELS

On effectue un test visuel de coloration de solutions phtochromiques selon l'invention.

Le test est effectué sur des solutions toluéniques de concentration 2,5 x 10⁻⁵ mole/1 dans des cuves en quartz.

On irradie les échantillons dans les mêmes conditions que pour la mesure des paramètres thermocinétiques.

On prend comme référence deux composés de l'art antérieur EP 561 915 :
Le composé A' de formule 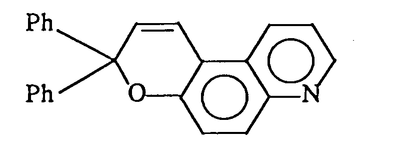
Le composé B' de formule

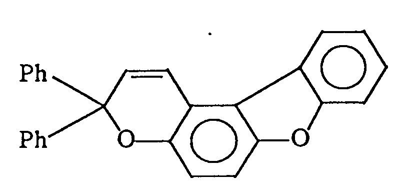

On compare les échantillons des composés photochromiques selon l'invention aux composés A' et B'.

On apprécie visuellement l'intensité de la coloration après irradiation :
- +: correspond à la coloration prise par les composés de référence,
- ++: correspond à une coloration plus intense que les composés en référence,
- +++: correspond à une coloration nettement plus intense que les composés de référence.

| Composé selon l'invention des exemples 1 à 7 | Couleur perçue | Intensité |
|---|---|---|
| 1 | rouge | +++ |
| 2 | rouge foncé | ++ |
| 3 | rouge rosé | +++ |
| 4 | rouge foncé | +++ |
| 5 | rouge foncé | +++ |
| 6 | rouge orangé | ++ |
| 7 | rouge rosé | +++ |
| Composés de référence | | |
| A' | orange | + |
| B' | rouge | + |

## Revendications

1. Composé photochromique répondant à la formule générale (I) : 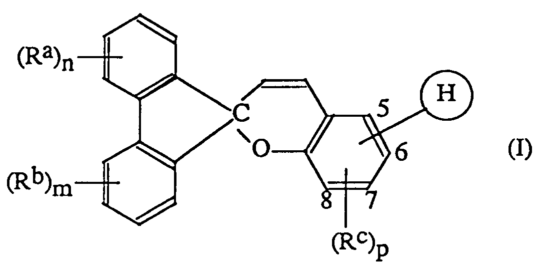 dans laquelle R^{a}, R^{b} et R^{c} désignent, indépendamment l'un de l'autre, un atome d'hydrogène, un groupement alkyle, un groupement aryle, un groupement OR, SR, COR ou COOR, dans lesquels R désigne un atome d'hydrogène, un groupement alkyle ou un groupement aryle, un groupement amino de formule NR₁R₂, dans lequel R₁ et R₂ désignent, indépendamment l'un de l'autre, un atome d'hydrogène, un groupement alkyle, un groupement cycloalkyle, un groupement aryle, R₁ et R₂ pouvant former avec l'atome d'azote, un hétérocycle comportant 4 à 7 chaînons et pouvant contenir en plus un ou plusieurs hétéroatomes choisis parmi l'azote, l'oxygène, le soufre, un atome d'halogène, un groupement mono ou polyhaloalkyle; un groupement NO₂, CN ou SCN; n et m désignent des nombres entiers de 1 à 4 suivant le nombre de substitutions sur le noyau et p peut être égal à 1 ou 2 suivant le nombre de substitutions sur le noyau; les groupements R^{a}, R^{b}, R^{c} peuvent avoir des significations différentes lorsque m, n et p sont supérieurs à 1 et suivant la position sur les noyaux; H est un hétérocycle aromatique à 5 ou 6 chaînons, condensé en 5,6 ou 6,7, comportant un ou plusieurs hétéroatomes choisis parmi l'oxygène, le selenium, le soufre ou l'azote ou un carbocycle aromatique à 5 ou 6 chaînons, condensé en 7,8; ledit carbocycle ou hétérocycle H pouvant être substitué par un ou plusieurs groupements alkyle, alcoxy, amino, aryle, aralkyle ou condensé avec un autre cycle de 5 à 10 chaînons.

2. Composé selon la revendication 1, caractérisé par le fait que dans la formule (I), le radical alkyle est en C₁-C₆; le cycloalkyle comporte 3 à 7 atomes de carbone; le groupe aryle est phényle; l'halogène désigne chlore, fluor, brome; le groupe polyhaloalkyle désigne CF₃.

3. Composé selon la revendication 1 ou 2, caractérisé par le fait que le carbocycle aromatique H condensé en position 7,8 est un cycle benzénique.

4. Composé selon la revendication 1 ou 2, caractérisé par le fait que l'hétérocycle H condensé en 5,6 ou 6,7 est choisi parmi ceux de formules : 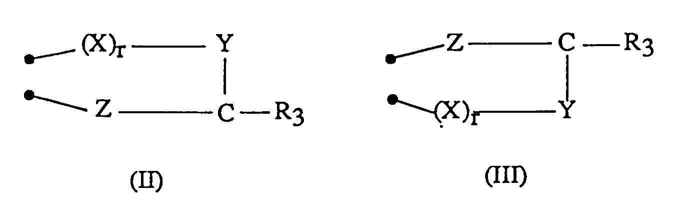 dans lesquelles :
X désigne N ou le groupe CR₄, l'atome d'azote ou de carbone étant relié à l'atome adjacent par une double liaison pour assurer l'aromaticité du cycle condensé sur le noyau benzopyrane de la formule (I);
r vaut 0 ou 1;
Y et Z, indépendamment l'un de l'autre, désignent NR₅, S, O, Se; ou bien N ou CR₆, chacun étant lié à l'atome adjacent par une double liaison pour assurer l'aromaticité de l'hétérocycle condensé sur le noyau benzopyrane de formule (I).
R₅ et R₆, désignent indépendamment l'un de l'autre, un atome d'hydrogène, un alkyle en C₁-C₆ ou un phényle;
CR₃ est lié à un atome adjacent par une double liaison de manière à assurer l'aromaticité de l'hétérocycle condensé sur le noyau benzopyrane de la formule (I);
R₃ désigne un atome d'hydrogène, un alkyle en C₁-C₆, un phényle ou forme avec l'un des groupes R₅ ou R₆ un cycle C, aromatique ou non, ayant de 5 à 10 chaînons, de préférence de 6 à 7 chaînons, éventuellement substitué par un ou plusieurs radicaux R^{a} tels que définis dans la revendication 1;
l'un des groupements X, Y et Z désigne N, O, S, Se ou NR₅.

5. Composé selon la revendication 4, caractérisé par le fait que dans la formule (II) ou (III), le cycle C désigne benzène, cyclohexane ou cycloheptane.

6. Composé selon l'une quelconque des revendications 1 à 5, caractérisé par le fait que l'hétérocycle H condensé en 6,7 ou 5,6 est choisi parmi le benzofurane, le cycloheptanofurane, le cyclohexanofurane, les noyaux pyrimidiques, pyraziniques, pyrroliques, pyridiniques, oxazoliques ou thiazoliques, éventuellement substitués.

7. Composé selon l'une quelconque des revendications 1 à 6, caractérisé par le fait qu'il comporte un hétérocycle H à 5 chaînons condensé en position 5,6 ou 6,7 sur le noyau benzopyrane de la formule (I).

8. Composé selon la revendication 7, caractérisé par le fait qu'il comporte un hétérocycle H à S chaînons condensé en position 6,7 sur le noyau benzopyrane de la formule (I).

9. Composé selon l'une quelconque des revendications 1 à 7, caractérisé par le fait qu'il est choisi parmi ceux de formules :

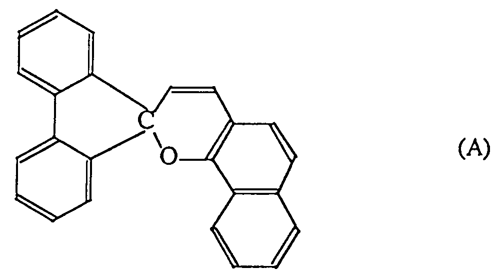

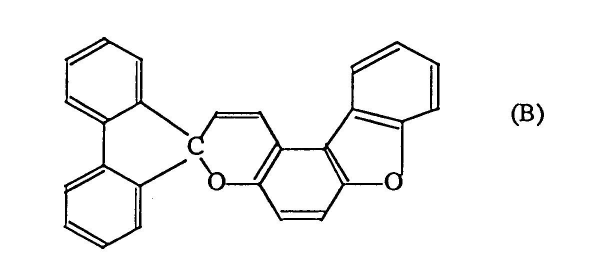

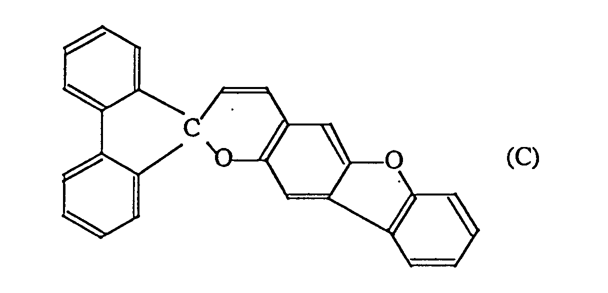

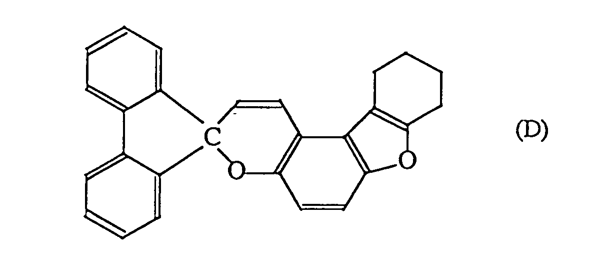

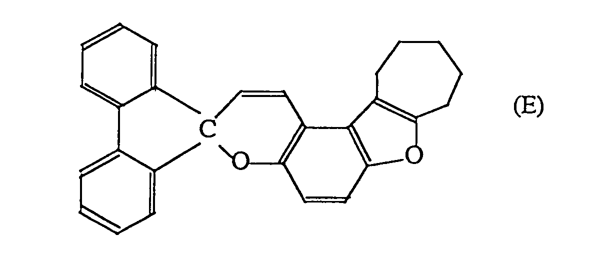

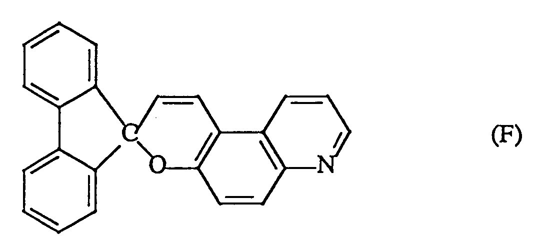

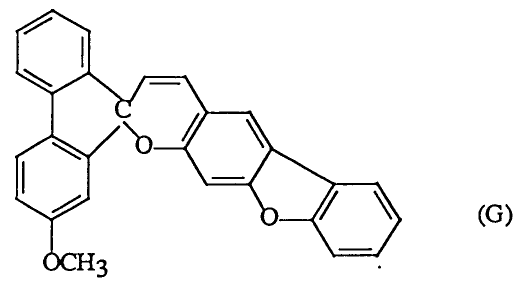

10. Utilisation du composé selon l'une quelconque des revendications 1 à 9, comme composé photochromique dans l'optique ophtalmique.

11. Composition destinée à être appliquée sur ou introduite dans un matériau polymère organique transparent, caractérisée par le fait qu'elle contient au moins un composé photochromique tel que défini dans l'une quelconque des revendications 1 à 9, dans des quantités suffisantes pour permettre au matériau exposé à une radiation ultraviolette de changer de couleur.

12. Composition selon la revendication 11, caractérisée par le fait que la composition est sous forme liquide contenant sous forme dissoute ou dispersée, les composés photochromiques selon l'une quelconque des revendications 1 à 9, dans un milieu à base de solvants appropriés pour être appliqués ou introduits dans un matériau polymère transparent.

13. Composition destinée à être appliquée sur ou introduite dans un matériau polymère organique transparent, caractérisée par le fait qu'elle est constituée par une solution incolore ou transparente à base de polymères, de copolymères ou de mélange de polymères transparents dans un solvant organique approprié, contenant au moins un composé photochromique tel que défini dans l'une quelconque des revendications 1 à 9, dans des quantités suffisantes pour permettre au matériau exposé à une radiation ultraviolette de changer de couleur.

14. Matériau solide transparent approprié pour réaliser des lentilles ophtalmiques, caractérisé par le fait qu'il comporte sur la surface et/ou à l'intérieur au moins un composé photochromique tel que défini dans l'une quelconque des revendications 1 à 9, dans des quantités suffisantes pour permettre au matériau exposé à une radiation ultraviolette de changer de couleur.

15. Matériau solide transparent selon la revendication 14, caractérisé par le fait qu'il contient 0,01 à 20% en poids de composés photochromiques.

16. Composition ou matériau solide transparent, selon l'une quelconque des revendications 11 à 15, caractérisée par le fait que le composé photochromique tel que défini dans l'une quelconque des revendications 1 à 9, est utilisé conjointement avec d'autres composés photochromiques donnant lieu à des colorations différentes.

17. Vernis de transfert, caractérisé par le fait qu'il contient au moins un composé tel que défini dans l'une quelconque des revendications 1 à 9.

18. Lentille ophtalmique, caractérisée par le fait qu'elle est réalisée à partir d'un matériau solide transparent tel que défini dans la revendication 14 ou 15.
